# EUROPÄISCHE PATENTSCHRIFT

(11) **EP 0 479 984 B1**
(45) Veröffentlichungstag und Bekanntmachung des Hinweises auf die Patenterteilung: **10.07.1996**
(21) Anmeldenummer: 91907550.7
(22) Anmeldetag: 22.04.1991
(51) Int. Cl.: G01N 33/36

(54) **TEXTILE ON-LINE-STICHPROBENANALYSE**
TEXTILE ON-LINE SAMPLING INSPECTION
ECHANTILLONNAGE DE TEXTILES EN LIGNE

(30) Priorität: 26.04.1990 CH 1436/90
(43) Veröffentlichungstag der Anmeldung: 15.04.1992
(73) Patentinhaber: ZELLWEGER LUWA AG, CH-8610 Uster (CH)
(72) Erfinder: TOEDTLI, Sergej, CH-8832 Wollerau (CH)
(86) Internationale Anmeldenummer: CH9100093
(87) Internationale Veröffentlichungsnummer: WO9116625

(56) Entgegenhaltungen:
- EP-A- 0 247 420
- GB-A- 1 315 958
- US-A- 3 669 552
- US-A- 4 295 252

## Beschreibung

Die Erfindung bezieht sich auf ein Verfahren zur stichprobenweisen Analyse von textilen Fasersträngen, gemäss dem Oberbegriff des Anspruchs 1.

Als Faserstränge im Sinne des erfindungsgemässen Verfahrens sollen geordnete und nicht geordnete Stränge von textilen Einzelfasern (Faserbänder), aber auch Vliese verstanden werden.

In der Textilindustrie ist es bekannt bei kontinuierlich bewegten Fasersträngen zur Qualitätsüberwachung mit mechanischen oder kapazitiven Sensoren die Massenschwankungen in den bewegten Fasersträngen oder Bändern automatisch zu messen. Fremdpartikel und Fremdfasern hingegen werden manuell gemessen, indem ein Laborant stichprobenweise Faserbänder auf einem Leuchttisch auseinanderzieht und von Auge die Fremdpartikel sucht und auszählt. Was die Eigenschaften der Einzelfasern betrifft, so können diese überhaupt nicht gemessen werden, was eine Feststellung von Parameteränderungen (z.B. Länge, Kräuselung) infolge der Bearbeitungsprozesse verunmöglicht.

Hier will die Erfindung Abhilfe schaffen. Der Erfindung liegt die Aufgabe zugrunde, ein Verfahren zur stichprobenweisen Analyse von textilen Fasersträngen zu schaffen, welches eine zerstörungsfreie Analyse am laufenden Faserstrang erlaubt.

Die Erfindung löst die gestellte Aufgabe mit einem Verfahren, welches die Merkmale des Anspruchs 1 aufweist, sowie einer Vorrichtung zur Durchführung des Verfahrens, welche die Merkmale des Anspruchs 5 aufweist.

Die durch die Erfindung erreichten Vorteile sind im wesentlichen darin zu sehen, dass dank des erfindungsgemässen Verfahrens eine "on-line" Analyse mittels Stichproben am laufenden Faserstrang (z.B. Kardenband) ohne bleibende Schädigung des Faserstranges ermöglicht wird.

Weitere Vorteile liegen in der automatischen Analyse von Fremdpartikeln und Erfassung von Längenänderungen infolge von Bearbeitungsprozessen. Diese Werte können auch für die Optimierung der vorangegangenen Bearbeitung verwendet werden. Die bei solchen Bearbeitungsprozessen routinemässig vorkommende Doublierung der Faserbänder kann kontrolliert und gesteuert werden, so dass die Werte der Faserparameter und damit auch die Garnqualität konstant bleiben.

Ein Ausführungsbeispiel der Erfindung, welches zugleich das Funktionsprinzip erläutert, ist in der Zeichnung dargestellt und wird im folgenden näher beschrieben.
Fig. 1 zeigt eine schematische Darstellung einer Vorrichtung zur Durchführung des erfindungsgemässen Verfahrens;
Fig. 2 zeigt eine graphische Darstellung der faserstrangeinebnenden Mittel der erfindungsgemässen Vorrichtung nach Fig. 1 vor ihrer Betätigung;
Fig. 3 zeigt eine graphische Darstellung der faserstrangeinebnenden Mittel der erfindungsgemässen Vorrichtung nach Fig. 1 nach dem Erfassen des Faserbandes;
Fig. 4 zeigt eine graphische Darstellung der faserstrangeinebnenden Mittel der erfindungsgemässen Vorrichtung nach Fig. 1 nach dem Auseinanderziehen des Faserbandes;
Fig. 5 zeigt eine Variante der Detaildarstellung nach Fig. 4, bei der Messung;
Fig. 6 zeigt typische Störpartikel in einem Baumwollfaservlies, welche durch das erfindungsgemässe Verfahren analysiert werden können; und
Fig. 7 zeigt eine Vielzahl von zentral gesteuerten, erfindungsgemässen Vorrichtungen zur stichprobenweisen, gleichzeitigen textilen Analyse mehrerer Faserstränge mittels verschiedenartiger Analyseverfahren.

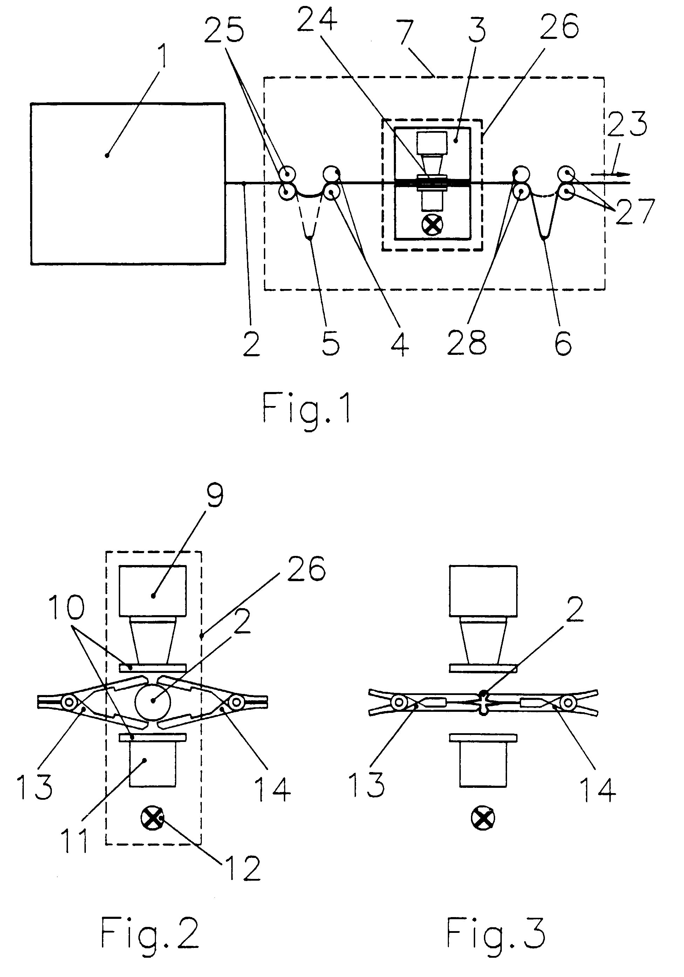

Die in Fig. 1 dargestellte Messapparatur 7 zur Durchführung des erfindungsgemässen Verfahrens besteht im wesentlichen aus faserstrangtransportierenden Mitteln 25,4,28,27, faserstrangstauenden Mitteln 5,6, faserstrangeinebnenden Mitteln 3 und einer textilen Messeinrichtung 26. Sie kann direkt, on-line an eine einen Faserstrang oder ein Faserband 2 produzierende Einheit 1, beispielsweise einem Streckwerk, einer Karde oder einer Kämmaschine angeschlossen werden.

Der links im Bild in die Messapparatur 7, mit einer Geschwindigkeit von 8 bis 120 m/min, eintretende Faserstrang 2 läuft zwischen zwei im Abstand von 0,1 - 1,0 m angeordneten, zusammenwirkenden Walzenpaaren 25,25;4,4 hindurch, gelangt dann in den Bereich der textilen Messeinrichtung 26 und verlässt schliesslich nach Durchtritt zwischen zwei im Abstand von 0,1 - 1,0 m angeordneten, zusammenwirkenden Walzenpaaren 28,28;27,27 die Messapparatur 7 in longitudinaler Richtung 23. Material, Oberfläche, Dimension und elektromotorischer Antrieb und Steuerung der Walzenpaare entsprechen vorzugsweise denjenigen von Standard-Streckwerken.

Durch kurzzeitiges Anhalten der Walzenpaare 4 und 28 bei weiterrotierenden Walzenpaaren 25 und 27 kann der Faserstrang 2 temporär innerhalb des von den Walzenpaaren 4 und 28 begrenzten Abschnitts zum Stillstand gebracht werden, wobei der Faserstrang 2 im Stauraum 5 (zwischen den Walzenpaaren 25 und 4) kurzfristig aufgestaut wird und die im vorhergehenden Ruhezyklus im Stauraum 6 (zwischen den Walzenpaaren 28 und 27) gebildete Stauung wieder abgetragen wird.

Zwischen den beiden Stauräumen 5 und 6 gelangt der Faserstrang 2 auf seinem Transportweg zwischen faserstrangeinebnende Mittel 3, welche in Fig. 2 durch die Platten 10 einer optischen Textil-Messeinrichtung 26 dargestellt sind. Die gegeneinander beweglich angeordneten Platten 10 gestatten ein kurzzeitiges - mit den zwischen den Walzenpaaren 25,4 und 28,27 stattfindenden Stauzyklen des Faserstranges 2 in den Stauräumen 5 und 6 synchronisiertes - Zusammendrücken des Faserstranges 2 im einem Teilbereich 24 des begrenzten, ruhenden Abschnitts 4,28. Dabei wird das Profil des Faserstranges 2 soweit eingeebnet, dass es einer diskontinuierlichen, textilen Prüfung, beispielsweise optischer Art, mittels der textilen Messeinrichtung 26 zugänglich wird. Vorzugsweise erfolgt die Einebnung des Faserstrangprofils 2 zu einer einlagigen Anordnung der Einzelfasern.

Die textile Messeinrichtung 26 ist vorzugsweise eine auf der Bilddatenverarbeitung beruhende Vorrichtung, welche in Fig. 2 durch eine Beleuchtungsquelle 12, eine Beleuchtungsoptik 11, eine Abbildungsoptik und eine Kamera 9 angedeutet ist.

Es können aber auch andere Messeinrichtungen 26 eingesetzt werden, z.B. mit anderen Optiken, mit Integralmessung oder mit Auflicht-(Reflexions)-Messung.

Nach durchgeführter Messung der textilen Parameter mittels der textilen Messeinrichtung 26 wird der temporär eingeebnete Teilbereich 24 des Faserstranges 2 wieder losgelassen und der kurzfristig zum Stillstand gebrachte, von den Walzenpaaren 4 und 28 begrenzte Abschnitt des Faserstranges 2 durch erneutes Rotierenlassen der Walzenpaare 4 und 28 weiter befördert, wobei der kurzfristig deformierte Teilbereich 24 regenerieren kann, so dass der Faserstrang 2 insgesamt keine vom Messverfahren herrührende bleibende Schädigung erfährt.

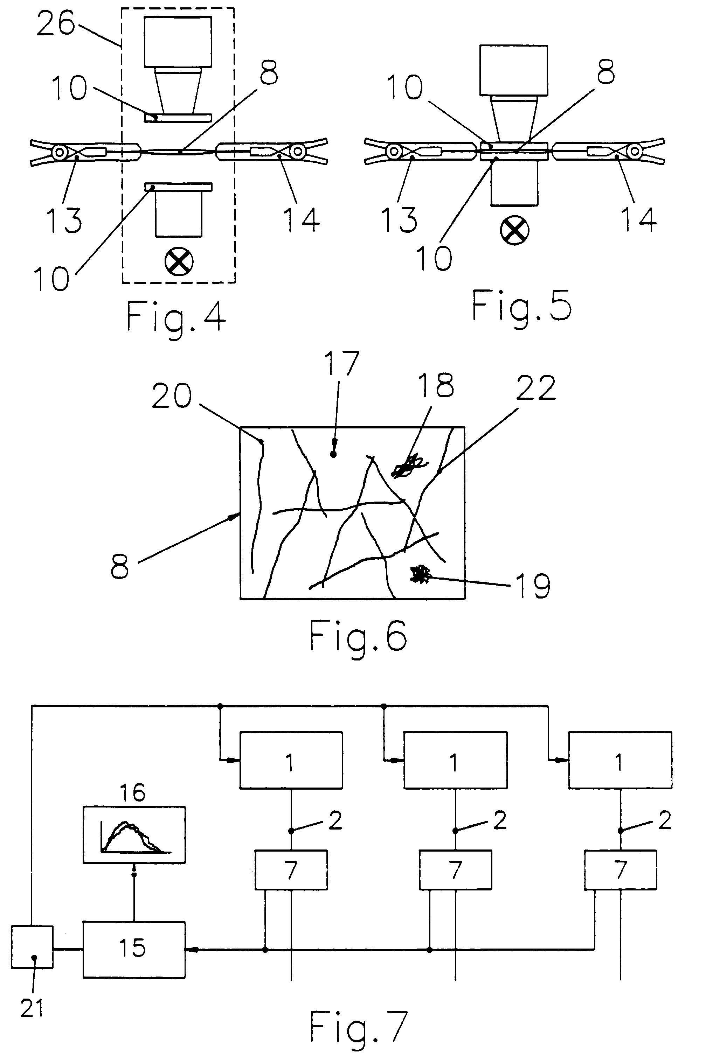

Das Einebnen des Faserstrangprofils 2 kann ausser der anhand der Fig. 1 beschriebenen Methode auch mittels einer speziellen Vorrichtung, wie in Fig. 2 - 4 dargestellt erfolgen. Dabei wird der Faserstrang 2, wie in Fig. 3 dargestellt, während seines Stillstands im Bereich der textilen Messeinrichtung 26 von einem lateral angeordneten Zangenpaar 13, 14 erfasst und eingeklemmt und wie in Fig. 4 dargestellt, durch Auseinanderziehen des Zangenpaares 13,14 zu einem durchsichtigen, vorzugsweise einlagigen Vlies 8 eingeebnet.

In den Fig. 4 und 5 ist weiter dargestellt, wie ein durchsichtige Baumwollvlies 8 während seines Stillstandes im Bereich der textilen Messeinrichtung 26 vom lateral angeordneten Zangenpaar 13, 14 an seinen Rändern gefasst und durch Zuganwendung weiter abgeflacht wird, so dass ein einlagiges Vlies entsteht. Gleichzeitig werden die kreisringförmigen Platten 10 der Beleuchtungsoptik 11 und der Abbildungsoptik 9 der textilen Messeinrichtung 26 gegeneinander gedrückt, so dass das vom Zangenpaar 13,14 festgeklemmte und auseinandergezogene Faservlies 8 weiter abgeflacht wird und die auf einer Bilddatenverarbeitung beruhende optische Textilprüfung durchgeführt werden kann, welche eine dünne, planare Anordnung des zu analysierenden Fasergutes voraussetzt.

In Fig. 6 ist ein Bildausschnitt dargestellt, wie er von der Abbildeoptik 9, der auf einer Bilddatenverarbeitung beruhenden, textilen Messeinrichtung 26 erzeugt wird; er zeigt typische Störpartikel in einem Baumwollfaservlies 8, beispielsweise in Form von Schmutz 17, Nissen 18, Schalenteilchen mit Fasern ("seed coat fragments") 19, Faserenden 20 und Fasern 22, welche durch das erfindungsgemässe Verfahren analysiert werden können. Die Methodik zur Erkennung solcher Störpartikel und die Bestimmung des relativen und absoluten Anteils der diversen Störpartikel ist bekannt und beispielsweise in Melliand Textilberichte 12/1989, Seite 887-889 beschrieben.

In Fig. 7 ist eine Vielzahl von einen Faserstrang 2 produzierenden Mitteln 1 mit je einer erfindungsgemässen Messapparatur 7 dargestellt, welche von einem einzigen, zentralen Rechner 15 gesteuert werden.

Die Parameter eines Faserbandes ändern sich relativ langsam, auch wenn aufgrund des Messergebnisses in den einen Faserstrang 2 produzierenden Mitteln 1 eine Veränderung vorgenommen wurde. Die Messtechnik ist erheblich schneller, so dass mehrere Bänder von einem Rechner 15 gleichzeitig überwacht werden können. Es können damit auch Bänder miteinander verglichen werden. Bei grösseren Abweichungen ist es möglich einen Alarm auszulösen. Es kann zudem auch eine weitere Optimierung erfolgen, indem Vorschläge für die Doublierung von Faserbändern unterschiedlicher Qualität ausgegeben, bzw. direkt veranlasst werden.

Die vom Rechner 15 erfassten Daten können auf einem optischen Anzeigegerät 16 graphisch oder anderweitig dargestellt werden und/oder über ein Interface 21 zur Steuerung der faserstrangproduzierenden Einheiten 1 benutzt werden.

Die Häufigkeit der Faserenden im Bildausschnitt lassen auf die Länge der Fasern schliessen. Ist diese Länge geringer als im Ausgangsmaterial, so müssen die einen Faserstrang 2 produzierenden Mittel 1 die Ursache davon sein. Über das Interface 21 kann dies der entsprechenden Maschine 1 mitgeteilt werden, die dann ihre Parameter korrigiert bis die Faserschädigung optimal klein ist. Der Rechner 15 verteilt diese Information an die entsprechenden Maschinen 1. Weitere Möglichkeiten dieser Art sind in der Internationalen Patentanmeldung WO 90/15325 "Verfahren zur Frequenzanalyse an bewegten Faserkollektiven" beschrieben.

## Patentansprüche

1. Verfahren zur stichprobenweisen Analyse von textilen Fasersträngen, dadurch gekennzeichnet, dass ein kontinuierlich in longitudinaler Richtung (23) bewegter Faserstrang (2) diskontinuierlich innerhalb eines begrenzten Abschnitts (4, 28) zum Stillstand gebracht wird, dass das Profil des ruhenden Abschnitts (4, 28) des Faserstranges (2) mindestens in einem Teilbereich (24) zerstörungsfrei eingeebnet wird und dass an diesem temporär ruhenden, eingeebneten Teilbereich (24) des Faserstranges (2) eine zerstörungsfreie textile Analyse durchgeführt wird.

2. Verfahren nach Anspruch 1, dadurch gekennzeichnet, dass der Teilbereich (24) derart eingeebnet wird, dass ein einlagiges Vlies entsteht.

3. Verfahren nach Anspruch 1 oder 2, dadurch gekennzeichnet, dass der Faserstrang (2) mit einer Geschwindigkeit von 8 - 120 m/min kontinuierlich in longitudinaler Richtung transportiert wird.

4. Verfahren nach einem der Ansprüche 1 bis 3, dadurch gekennzeichnet, dass der Faserstrang (2) innerhalb des begrenzten Abschnitts (4,28) mit einer Frequenz von 2 bis 60 Mal pro Minute zum Stillstand gebracht wird.

5. Vorrichtung zur Durchführung des Verfahrens nach einem der Ansprüche 1 bis 4 mit faserstrangtransportierenden Mitteln (25, 4, 28, 27), faserstrangstauenden Mitteln (5, 6), faserstrangeinebnenden Mitteln (3) und einer zerstörungsfreien textilen Messeinrichtung (26).

6. Vorrichtung nach Anspruch 5, dadurch gekennzeichnet, dass die faserstrangtransportierenden Mitteln (25,4,28,27) zwei, vor der Messeinrichtung (26) angeordnete, zusammenwirkende Walzenpaare (25,25;4,4) und zwei, nach der Messeinrichtung (26) angeordnete, zusammenwirkende Walzenpaaren (28,28;27,27) umfassen, welche vorzugsweise über einen elektromotorischen Antrieb verfügen.

7. Vorrichtung nach Anspruch 6, dadurch gekennzeichnet, dass die zwei, vor der Messeinrichtung (26) angeordneten Walzenpaare (25,25;4,4) einen gegenseitigen Abstand von vorzugsweise 0,1 - 1,0 m aufweisen, der einen Stauraum (5) für den Faserstrang (2) bildet.

8. Vorrichtung nach Anspruch 6, dadurch gekennzeichnet, dass die zwei, nach der Messeinrichtung (26) angeordneten Walzenpaare (28,28;27,27) einen gegenseitigen Abstand von vorzugsweise 0,1 - 1,0 m aufweisen, der einen Stauraum (6) für den Faserstrang (2) bildet.

9. Vorrichtung nach einem der Ansprüche 5 bis 8, dadurch gekennzeichnet, dass die faserstrangeinebnenden Mittel (3) zwei auf entgegengesetzten Seiten des Faserstranges (2) angeordnete, beweglich zusammenwirkende, parallele Platten (10) einer optischen Textil-Messeinrichtung (26) umfassen.

10. Vorrichtung nach einem der Ansprüche 5 bis 8, dadurch gekennzeichnet, dass die faserstrangeinebnenden Mittel (3) ein lateral auf entgegengesetzten Seiten des Faserstranges (2) angeordnetes, bewegliches Zangenpaar (13, 14) umfassen.

11. Vorrichtung nach einem der Ansprüche 5 bis 10, dadurch gekennzeichnet, dass sie von einem zentralen Rechner (15) überwacht und gesteuert wird, der an eine Vielzahl von solchen Vorrichtungen angeschlossen ist.

12. Vorrichtung nach Anspruch 11, dadurch gekennzeichnet, dass die vom Rechner (15) erfassten Daten auf einem optischen Anzeigegerät (16) graphisch, optisch oder akustisch dargestellt werden.

13. Vorrichtung nach Anspruch 11 oder 12, dadurch gekennzeichnet, dass die vom Rechner (15) erfassten Daten über ein Interface (21) zur Steuerung der faserstrangproduzierenden Einheiten (1) benutzt werden.

## Claims

1. Method for the random-sample analysis of textile fibre strings, characterized in that a fibre string (2) moved continuously in the longitudinal direction (23) is brought to a standstill discontinuously within a limited section (4, 28), in that the profile of the stationary section (4, 28) of the fibre string (2) is levelled non-destructively at least in a part region (24), and in that a non-destructive textile analysis is carried out on this temporarily stationary levelled part region (24) of the fibre string (2).

2. Method according to Claim 1, characterized in that the part region (24) is levelled in such a way that a single-layer web is obtained.

3. Method according to Claim 1 or 2, characterized in that the fibre string (2) is transported continuously in the longitudinal direction at a speed of 8 - 120 m/min.

4. Method according to one of Claims 1 to 3, characterized in that the fibre string (2) within the limited section (4,28) is brought to a standstill at a frequency of 2 to 60 times per minute.

5. Apparatus for carrying out the method according to one of Claims 1 to 4, with fibre-string transporting means (25, 4, 28, 27), fibre-string building-up means (5, 6), fibre-string levelling means (3) and a non-destructive textile measuring device (26).

6. Apparatus according to Claim 5, characterized in that the fibre-string transporting means (25,4,28,27) comprise two cooperating pairs of rollers (25,25;4,4), arranged upstream of the measuring device (26), and two cooperating pairs of rollers (28,28;27,27), arranged downstream of the measuring device (26), the said pairs of rollers preferably having an electromotive drive.

7. Apparatus according to Claim 6, characterized in that the two pairs of rollers (25,25;4,4) arranged upstream of the measuring device (26) are at a mutual distance of preferably 0,1 - 1.0 m which forms a build-up space (5) for the fibre string (2).

8. Apparatus according to Claim 6, characterized in that the two pairs of rollers (28,28;27,27) arranged downstream of the measuring device (26) are at a mutual distance of preferably 0.1 - 1.0 m which forms a build-up space (6) for the fibre string (2).

9. Apparatus according to one of Claims 5 to 8, characterized in that the fibre-string levelling means (3) comprise two movably cooperating parallel plates (10) of an optical textile-measuring device (26) which are arranged on opposite sides of the fibre string (2).

10. Apparatus according to one of Claims 5 to 8, characterized in that the fibre-string levelling means (3) comprise a movable pair of tongs (13, 14) arranged laterally on opposite sides of the fibre string (2).

11. Apparatus according to one of Claims 5 to 10, characterized in that it is monitored and controlled by a central computer (15) which is connected to a multiplicity of such apparatuses.

12. Apparatus according to Claim 11, characterized in that the data recorded by the computer (15) are displayed graphically, visually or acoustically on a visual indicator (16).

13. Apparatus according to Claim 11 or 12, characterized in that the data recorded by the computer (15) are used via an interface (21) for controlling fibre-string producing units (1).

## Revendications

1. Procédé d'échantillonnage d'écheveaux de fibres textiles, caractérisé en ce qu'un écheveau de fibres (2) se déplaçant continuellement dans la direction longitudinale (23) est arrêté de manière discontinue à l'intérieur d'un tronçon limité (4,28), en ce que le profil du tronçon en repos (4,28) de l'écheveau de fibres (2) est nivelé sans endommagement au moins dans une zone partielle (24) et en ce qu'on procède à une analyse textile non destructrice de cette zonepartielle nivelée (24), temporairement au repos, de l'écheveau de fibres (2).

2. Procédé selon la revendication 1, caractérisé en ce que la zone partielle (24) est nivelée de façon à obtenir une nappe en une couche.

3. Procédé selon la revendication 1 ou 2, caractérisé en ce que l'écheveau de fibres (2) est transporté à une vitesse de 8 - 120 m/mn continuellement dans la direction longitudinale.

4. Procédé selon l'une des revendications 1 à 3, caractérisé en ce que l'écheveau de fibres (2) est arrêté à l'intérieur du tronçon limité (4,28) à une fréquence de 2 à 60 fois par minute.

5. Dispositif pour exécuter le procédé selon l'une des revendications 1 à 4, avec des moyens (25,4,28,27) transportant les écheveaux de fibres, des moyens (5,6) retenant les écheveaux de fibres, des moyens (3) nivelant les écheveaux de fibres et un dispositif de mesure textile (26) non destructeur.

6. Dispositif selon la revendication 5, caractérisé en ce que les moyens (25,4,28,27) transportant les écheveaux de fibres comprennent deux paires de rouleaux coopérants (25,25;4,4) disposés devant le dispositif de mesure (26) et deux paires de rouleaux coopérants (28,28;27,27) disposés derrière le dispositif de mesure (26) qui disposent de préférence d'un entraînement par moteur électrique.

7. Dispositif selon la revendication 6, caractérisé en ce que les deux paires de rouleaux (25,25;4,4) disposés devant le dispositif de mesure (26) ont un écart mutuel, de préférence de 0,1 - 1,0 m, qui constitue une enceinte de retenue (5) de l'écheveau de fibres (2).

8. Dispositif selon la revendication 6, caractérisé en ce que les deux paires de rouleaux (28,28;27,27) disposés derrière le dispositif de mesure (26) ont un écart mutuel, de préférence de 0,1 - 1,0 m, qui constitue une enceinte de retenue (6) de l'écheveau de fibres (2).

9. Dispositif selon la revendication 5 à 8, caractérisé en ce que les moyens de nivellement d'écheveaux de fibres (3) comportent deux plaques parallèles (10) disposées aux côtés opposés de l'écheveau de fibres (2), coopérant de façon mobile, d'un dispositif de mesure textile optique (26).

10. Dispositif selon l'une des revendications 5 à 8, caractérisé en ce que les moyens de nivellement des écheveaux de fibres (3) comportent une paire de pinces mobiles (13,14) disposées latéralement sur les côtés opposés de l'écheveau de fibres (2).

11. Dispositif selon l'une des revendications 5 à 10, caractérisé en ce qu'il est surveillé et commandé par un calculateur central (15) qui est raccordé à un grand nombre de tels dispositifs.

12. Dispositif selon la revendication 11, caractérisé en ce que les données saisies par le calculateur (15) sont représentées sur un appareil indicateur optique (16) sous forme graphique, optique ou acoustique.

13. Dispositif selon la revendication 11 ou 12, caractérisé en ce que les données saisies par le calculateur (15) sont utilisées par une interface (21) pour commander les unités (1) produisant les écheveaux de fibres.
